(19) Europäisches Patentamt
European Patent Office
Office européen des brevets

(11) Publication number: **0 253 314 B1**

(12) **EUROPEAN PATENT SPECIFICATION**

(45) Date of publication of patent specification: **15.01.92**

(51) Int. Cl.[5]: **C12N 9/60**, //(C12N9/60, C12R1:865)

(21) Application number: **87109928.9**

(22) Date of filing: **09.07.87**

(54) **Peptidase and process for producing the same.**

(30) Priority: **09.07.86 JP 161220/86**
**30.06.87 JP 163178/87**

(43) Date of publication of application:
**20.01.88 Bulletin 88/03**

(45) Publication of the grant of the patent:
**15.01.92 Bulletin 92/03**

(84) Designated Contracting States:
**CH DE FR GB IT LI NL SE**

(56) References cited:

**BIOCHEMICAL AND BIOPHYSICAL RESEARCH COMMUNICATIONS, vol. 145, no. 1, 29th May 1987, pages 316-322, Academic Press, Inc., Duluth, MN, US; T. OKADA et al.: "Novel Leu-Lys-specific peptidase (Leulysin) produced by gel-entrapped yeast cells"**

**JOURNAL OF BIOCHEMISTRY, vol. 82, no. 6, 1977, pages 1689-1693; T. TANAKA et al.: "Degradation of mating factor by alpha-mating type cells of Saccharomyces cerevisiae"**

(73) Proprietor: **Suntory Limited**
**1-40 Dojimahama 2-chome Kita-ku**
**Osaka-shi Osaka-fu 530(JP)**

(72) Inventor: **Okada, Tsutomo**
**38-2, Nishigoshonouchi-cho Kinugasa**
**Kita-ku Kyoto-shi Kyoto(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a novel peptidase which is secreted by yeast, more particularly, Saccharomyces yeast, and a process for production thereof. The peptidase of the present invention is useful as an enzyme to cleave the peptide bond specifically at the C-terminal side of a Leu residue of a peptide which has an amino acid sequence of ... Leu - Lys ... in its primary structure.

In recent years, specific processing enzymes which convert pre-pro-peptides, a translation product of mRNA, into mature peptides are attracting attention, in line with developments in genetic engineering which have been enabling microbiological preparation of various hormones of higher animals.

For example, J. Kurjan et al. (Cell 30, 933 - 943 (l982)) and D. Julius et al (Cell 32, 839 - 852 (l983)) reported membrane-bound dipeptidyl aminopeptidases which process a precursor polypeptide to produce α-mating factor (hereinafter abbreviated as α-MF) of yeast. These enzymes cleave the peptide bond at carboxyl side of an Ala residue in a recurring sequence of - Glu - Ala -. Also, D. Julius et al. reported the structure of a membrane-bound endopeptidase, an enzyme which processes pre-pro α-MF and the structure of a gene which codes for the enzyme (Cell 37, l075 - l089 (l984)). The enzyme cleaves the bond between Lys - Arg. Furthermore, Matsuo et al. reported an intracellular enzyme, Phorcesin Y-l, as an enzyme which activates pro-hormone of yeast (Nature (London) 309 (5968), 558 - 560 (l984) and Japanese Patent Public Disclosure No. 2l7894/l985). Its mechanism of action is to cleave the bond between Arg - Arg or Arg - Lys specifically. Other reported peptidases produced by yeast include carboxy-peptidase Y, proteinase A, proteinase B, aminopeptidase, carboxypeptidase S (H. Holzer, H. Bets and E. Ebner: Curr. Top, Cell. Regul., 9, l03 - l56 (l975)).

On the other hand, it has been revealed that α-MF is a peptide with a molecular weight of l684 whose primary structure is represented by the following amino acid sequence (I):

Trp - His - Trp - Leu - Gln - Leu ↑ Lys -
Pro - Gly - Gln - Pro - Met - Tyr (I)

and that it can be obtained by shake culture of α-type cell X-2l80-lB of S. cerevisiae in Burkholder medium. Also, it is known that the longer the culture period is, the greater is the variety of peptides other than α-MF that are formed in the medium, this being accompanied by a sharp decrease in α-MF activity in the medium. Tanaka et al. identified peptides which resulted from the cleavage of α-MF at the site marked with an arrow in (I) above, by analyzing various peptides formed after a long period of culture (J. Biochem., 82, l689 - l693 (l977)). The activity of the substance causing the cleavage, however, was very weak in all cases (only about 50% of α-MF produced at a level of several μg/ml was cleaved after being reacted for 24 hours). Besides, various side reactions were observed, and the identity of the substance has not yet been revealed. J. Thorner et. al. speculate that the observed α-MF cleaving activity results from a hormone peptidase secreted by the yeast extracellularly (J. Thorner, l98l, Phoremonal regulation of development in Saccharomyces cerevisiae. In the molecular biology of the yeast Saccharomyces. I. Life cycle and inheritance (edited by J. Strathern et al.), p. l43, Cold Spring Harbor Laboratory, Cold Spring Harbor, New York). However, its identity has not yet been clarified.

The present invention identifies the entity of activity for cleaving the bond between Leu - Lys in α-MF, and provides a novel peptidase which acts specifically on the bond between Leu - Lys, as well as a preparation procedure therefor.

The inventors of the present invention found that when the yeast cells are immobilized in a carrier at a high density (l - 2 g wet weight of cell/l g gel) for culture, the α-MF cleaving activity of the culture solution is much higher than that of a suspended cell culture solution. The present invention was completed on the basis of these findings.

Fig. l is a graph showing the elution process in column chromatography of a crude solution containing the enzyme of the present invention, using DEAE-Sephacel®;

Fig. 2 and Fig. 3 are each a graph showing the results of molecular weight determination of the enzyme of the present invention, performed by HPLC using a molecular sieve of TSK-Gel G3000SW or Sephacryl® S500;

Fig. 4 is a SDS-PAGE pattern of the enzyme of this invention;

Fig. 5 is a graph showing the temperature characteristics of the enzyme of the present invention;

Fig. 6 is a graph showing the pH characteristics of the enzyme of the present invention.

The Enzyme Properties

The enzyme properties of the present invention are as follows:

(l) Action: When acting on a peptide containing amino acid sequence of leucine - lysine (Leu - Lys), this enzyme specifically cleaves the peptide bond between Leu and Lys via hydrolysis.

(2) Substrate specificity: For example, this enzyme cleaves the bond in a peptide at the site marked with an arrow as in the following. That is, it cleaves Leu ↓ Lys bond in the molecule only at the C-terminal side of Leu residue; this enzyme does not hydrolyze any bonds when Lys and Leu are bound to other amino acids.

(3) Molecular weight: The molecular weight of this enzyme, estimated by silica gel resin and Sephacryl® resin molecular sieve chromatographies with the active form of the enzyme, is ca. l00,000, and ca. 750,000. The enzyme consists of subunits having ca. 55 kd and ca. 63 kd, respectively.

(4) The optimum pH and stable pH range: The optimum pH is ca. 4; when treated at 30 °C for 24 hours, this enzyme is stable over the pH range of 3 - 4.5.

(5) The range of optimum temperature for action: This enzyme acts over a temperature range of 20 °C - 60 °C and the optimum temperature range is 35 °C - 42 °C.

(6) Response to various inhibitors:

l. inhibited neither by phenylmethylsulfonyl fluoride (PMSF) nor by diisopropyl fluorophosphate (DFP), which are serine protease inhibitors, in a concentration of $2 \times l0^{-4}$ M.

2. inhibited neither by monoiodoacetic acid nor by p-chloro-mercuribenzoic acid (PCMB), which are thiol protease inhibitors.

3. not inhibited by EDTA which is a metal chelating agent.

4. inhibited neither by tosyl - L - lysine chloromethyl ketone (in a concentration of $l0^{-3}$ M) nor by leupeptin (in a concentration of $l0^{-4}$ M), which are typical trypsin inhibitors.

5. not inhibited by pepstatin (in a concentration of $l0^{-5}$ M) which is an acid protease inhibitor.

Procedure for Preparing the Enzyme

The peptidase according to the present invention can be obtained as follows: immobilize Saccharomyces yeast in an immobilizing carrier of high density, culture it for 24 hours and purify the enzyme from the culture solution.

The Saccharomyces yeast used in the present invention is preferably an α-mating type yeast, but other yeasts can also be used as long as they produce the peptidase described in the present invention; for example, Saccharomyces cerevisiae, more specifically, X2l80-lB strain (ATCC 26787) which is a representative haploid strain of Saccharomyces cerevisiae can be used.

The yeast mentioned above is pre-cultured, and the obtained yeast cells are collected and immobilized in a carrier. Any culture medium can be used as long as it allows the said yeast to grow and produce the peptidase.

A carbon source such as, e.g., glucose, dextrin, or treacle can be used; the most preferable being glucose. A nitrogen source such as, e.g., protein decomposition product (e.g. peptone, casamino acid, etc.), meat extract, yeast extract, amino acids, or ammonium salts can be used; it being preferable to use amino acids or ammonium salts. Also, vitamins and nucleic acid related compounds can be added for the purpose of promoting the growth of yeast. In pre-culture, it is preferable to use liquid medium in a spinner culture with aeration or in a shaking culture, for the purpose of obtaining a large amount of yeast cells, although a solid medium can also be used. Such culture conditions as temperature, duration, liquid characteristics of the medium, etc. are selected and controlled appropriately so that the yeast cell growth becomes maximum; generally, it is preferable to culture at 30 °C for 24 - 30 hours under aerobic conditions. The yeast cells cultured as described above are collected and immobilized according to a routine method.

Any type of carrier for immobilization is acceptable as long as it allows the growth of yeast cells which are immobilized at a high density; natural polysaccharides such as κ-carrageenan, sodium alginate, etc., and synthetic prepolymers such as photo-crosslinkable resin (ENTl000 -6000), polyvinyl alcohol resin, etc. can be used. Of these, hydrophilic photo-crosslinkable resin ENT2000 is convenient for immobilizing the yeast cells at a high density. The density of the yeast cells should be l - 2 g in wet weight per l g of carrier. It is necessary to be careful because the desired enzyme cannot be obtained in a satisfactory amount if the yeast cell density is low. For example, when 200 mg of wet weight yeast cells is immobilized per l g of the carrier, the amount of enzyme produced is not more than l/50 of the amount when yeast cells are immobilized in the density range specified above.

The immobilization is conducted by a routine method; for example, when the hydrophilic photo-crosslinkable resin described above is to be used, add an accelerator for polymerization, e.g. 2 mg of benzoin ethyl ether, to l g of photo-crosslinkable prepolymer, mix them with the yeast cells, and irradiate the mixture with near ultra-violet light (300 - 400 nm). Immobilized yeast cells are thus obtained.

The yeast thus immobilized is cultured in the above-mentioned medium, preferably Burkholder synthetic medium, for 24 hours to allow the enzyme to be produced and the enzyme is secreted outside of the immobilized yeast cells. The enzyme related to the present invention is secreted outside of the immobilizing carrier so that it can be obtained in the culture solution.

Purification of the enzyme from the obtained culture solution is conducted by employing an appropriate combination of routine methods. For example, by centrifuging or filtering the culture solution, insoluble matter is removed, and after the resulting liquid has been concentrated by ultrafiltration, it is dialyzed against water or a buffer solution. The solution is then subjected to column chromatography for fractionation using adsorbent resin consisting of DEAE cellulose or DEAE Sephadex®, e.g. DEAE Sephacel, and fractions eluted with 0.l - 0.6 M sodium chloride solution preferably with 0.2 M sodium chloride solution, are collected. These fractions are subjected to molecular sieve chromatography, and fractions that contain a substance with a large molecular weight (ca. l00,000 or ca. 750,000) are collected, which give the peptidase of the present invention. The eluted solution is demineralized, if necessary and can be used as it is; it can also be processed into a dried product after routine methods such as vacuum drying, freeze-drying, etc.

## Determination of Enzymatic Activity

The activity of peptidase of the present invention was determined by measuring the amount of Trp - His - Trp -Leu - Gln - Leu peptide (hereinafter denoted as l - 6 peptide) which is formed in accordance with the cleavage of a bond between - Leu - Lys -, after the peptidase underwent an enzymatic reaction with synthesized α-MF (Trp - His - Trp - Leu - Gln - Leu - Lys - Pro - Gly - Gln - Pro - Met - Tyr) used as a substrate. For example, to 0.4 ml of 0.05 M acetate buffer (pH 4.0) containing l.0 mmoles (l.6 µg) of chemically synthesized α-MF as substrate, l0 µl of the test enzyme solution is added and the mixture is allowed to react for 24 hours at 30°C. The amount of cleaved product (Trp - His - Trp - Leu - Gln - Leu) formed during this period is determined by High Performance Liquid Chromatography (hereinafter abbreviated as HPLC).

HPLC can be conducted under the following conditions, for example:

column: µ Bondapak $C_{18}$ (Waters)

Eluant:

Solution A = 50 mM phosphate buffer containing 5% acetonitrile (pH 2.0)
Solution B = 50 mM phosphate buffer containing 60% acetonitrile (pH 2.0)

Elution Method: Elution is conducted by a linear gradient method in which the eluant is changed from l00% Solution A to l00% Solution B at a flow rate of l.5 ml/minute over 30 minutes .

Detection: 280 nm and 2l4 nm.

## Example l: Production and Purification of Enzyme, and Estimation of its Molecular Weight

The yeast *Saccharomyces cerevisiae* X2l80-lB was shaking-cultured in Burkholder medium for 28 hours at 30°C, followed by centrifuging at 3000 rpm for l0 minutes to give the wet yeast cells. l.5 g of these yeast cells were mixed with l g of hydrophilic photo-crosslinkable prepolymer ENT2000 (manufactured by Kansai Paint Co., Ltd.) and 2 mg of benzoin ethyl ether, an accelerator for polymerization, and the mixture was irradiated with near ultraviolet light (300 - 400 nm) for polymerization to give immobilized yeast cells. The immobilized yeast cells were cultured on Burkholder medium for 24 hours at 30°C. The activity of the culture solution to cleave α-MF was found to be l.5 µg α-MF/l ml/24 hours. The culture solution (400 ml) was filtered aseptically with a millipore filter with pore size 2.2 µm, and it was concentrated further using membrane PU-l0 (manufactured by Amicon Corp.) for ultrafiltration. The rate of filtration was ca. 25 ml/30 minutes with a filter of 43 mm in diameter, and ca. 50 ml/30 minutes with that of 60 mm in diameter.

The portion which did not pass the filters was further dialyzed against water using a cellophane tube. The inner fluid of the dialysis was subjected to centrifuge at l0,000 rpm for l0 minutes, followed by lyophilization to give a crude product as white powder. The enzymatic activity of the powder was stable for at least 3 months, and the aqueous solution of the powder exhibited similarly stability.

The lyophilized powder obtained as described above was dissolved in 4 ml of l0 mM Tris-HCl buffer (pH 8.2), 3.5 ml of the resulting solution was subjected to column chromatography (0.5 × 2.0 cm) using DEAE-Sephacel® (manufactured by Pharmacia), and elution was conducted step-wise with l0 mM Tris-HCl buffer solution (pH 8.2) containing NaCl at respective concentrations of 0.2, 0.4, 0.6, 0.8 and l.0 M (flow rate:

l ml/l5 minutes). The results are shown in Fig. l. The fractions eluted with 0.2 M NaCl in Fig. l were collected as the peptidase-active portion.

(A) The molecular weight of the enzyme of the present invention was determined by molecular sieve chromatography (0.75 × 60 cm) using a silica gel resin (TSK-Gel G3000SW manufactured by Toyo Soda Manufacturing Co., Ltd.): 0.l ml of the fraction purified with DEAE Sephacel® and eluted with 0.2 M NaCl, as obtained above, was subjected to molecular sieve chromatography, and eluted with 0.l M potassium phosphate buffer (containing 0.2 M NaCl, pH 7.0) (flow rate: 0.7 ml/minute, 280 nm 0.l0 OD). The result, was that the activity peak of the enzyme of the present invention was observed to have a retention time of 2l.2l minutes, which shows the molecular weight of the active form of the enzyme is estimated ca. l00,000 on the basis of the comparison with an elution profile (retention time) of known standard substances (Fig. 2).

(B) A concentrated peptidase solution produced by culture and concentration as in Example l-(A), was subjected to a DEAE-Sephacel® column and eluted with 0.2 M NaCl as described in Example l-(A) to collect fractions exhibiting activities. This procedure was repeated five times.

The thus obtained fractions were used to measure the molecular weight of the enzyme of the invention. The fractions were subjected to a molecular sieve chromatography (l × 45 cm) using Sephacryl® S-500, and eluted with 0.l M acetate buffer (pH 6.0) at a flow rate of 0.l5 ml/minute. The activity peak of the enzyme of the present invention was observed in a retention volume of 30 ml, which shows the molecular weight of the active form the enzyme to be ca. 750,000 (Fig. 3).

Although the estimated molecular weight determined by Sephacryl® S-500 chromatography differs from that estimated by the method of Example l-(A), it is believed that this was caused by the use of a different type of molecular sieve column and that the enzymes purified by either procedure are essentially identical. When using a silica gel column, the molecules would be adsorbed to lower the molecular weight.

The enzyme of this invention was analyzed by use of SDS-PAGE to determine its subunits. Fractions which were purified by using a Sephacryl® S-500 column, dialyzed, desalted and freeze-dried were used as the sample. The freeze-dried sample was dissolved in 60 mM tris-HCl buffer containing 2.3% SDS (pH 6.8), and the solution was subjected to SDS-PAGE with l3.6% polyacrylamide gel (l6 × l8 cm × 0.75 mm) at a current of 40 mA for 3 hours. The resulting protein bands were stained with coomassie brilliant blue R250. From the results, it was confirmed that the enzyme of this invention is a protein consisting of subunits having ca. 55 kd and ca. 63 kd in comparison with the standard proteins concurrently run.

Example 2: Enzyme Properties

(l) Optimum Temperature for Action

l0 µl of the fraction, purified with DEAE-Sephacel and eluted with 0.2 M NaCl was used. As a substrate, l.5 µg of chemically synthesized α-MF was used, and the rate of action of the enzyme was determined in 0.05 M acetate buffer (pH 4.0) at respective temperatures between 20 - 50 °C. The result showed that the optimum temperature is 35 - 42 °C (Fig. 5).

(2) Optimum pH for Action

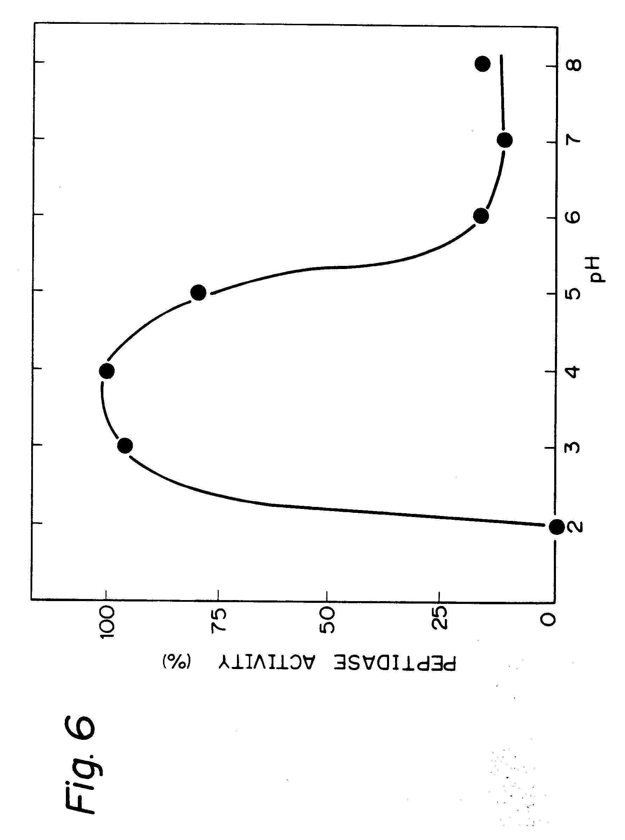

l0 µl of the fraction, purified by DEAE-Sephacel® and eluted with 0.2 M NaCl, was used. Using l.5 µg of chemically synthesized α-MF as a substrate, the rate of action of the enzyme was measured at 30 °C in 0.05 M acetate buffer solutions and 0.05 M phosphate buffer solutions with pH 2 - 8. The result showed that the optimum pH is 3 - 4.5 (Fig. 6).

(3) Substrate Specificity of Enzyme

The substrate specificity was analyzed by determining the percentage of cleaved substrate by HPLC, after allowing the fraction, purified with DEAE-Sephacel® chromatography and eluted with 0.2 M NaCl, to react with various substrates for 24 hours at 30 °C in 0.05 M acetate buffer (pH 4.0).

Peptides listed in Table l as:

l. those with Leu - Lys bond in the middle of the molecule,

2. those with Lys - X or X - Lys bond in the molecule, and

3. those with Leu - X or X - Leu bond in the molecule

(wherein X is an amino acid other than Leu in the case of 2 and is an amino acid other than Lys in the case of 3) were used as substrates. The results are shown in Table 2. The results showed that only those

peptides with Leu - Lys bond in the molecule which are classified in I above are cleaved, and that the enzyme of the present invention acts specifically on the Leu - Lys bond.

## Table 1

### Peptides used for testing reaction specificity of enzyme

| | | |
|---|---|---|
| 1. | α-MF (MW 1684.0) | Trp - His - Trp - Leu - Gln - Leu - Lys - Pro - Gly - Gln - Pro - Met - Tyr |
| | Dynorphin (MW 2147.5) | Trp - Gly - Gly - Phe - Leu - Arg - Arg - Ile - Arg - Pro - Lys - Leu - Lys - Trp - Asp - Asn - Gln |
| 2. | Neurotensin (MW 1672.9) | pGlu - Leu - Tyr - Glu - Asn - Lys - Pro - Arg - Arg - Pro - Tyr - Ile - Leu |
| | Tuftsin (MW 500.6) | Thr - Lys - Pro - Arg |
| 3. | β-Endorphin (MW 1859.1) | Tyr - Gly - Gly - Phe - Met - Thr - Ser - Glu - Lys - Ser - Gln - Thr - Pro - Leu - Val - Thr - Leu |
| | α-hANP (MW 3080.5) | Ser - Leu - Arg - Arg - Ser - Ser - Cys - Phe - Gly - Gly - Arg - Met - Asp - Arg - Ile - Gly - Ala - Gln - Ser - Gly - Leu - Gly - Cys - Asn - Ser - Phe - Arg - Tyr |
| | Cortitropin (CRF) (MW 4757.5) | Ser - Glu - Glu - Pro - Pro - Ile - Ser - Leu - Asp - Leu - Thr - Phe - His - Leu - Leu - Arg - Glu - Val - Leu - Glu - Met - Ala - Arg - Ala - Glu - Gln - Leu - Ala - Gln - Gln - Ala - His - Ser - Asn - Arg - Lys - Leu - Met - Glu - Ile - Ile - $NH_2$ |
| | Oxytocin (MW 1007.5) | Cys - Tyr - Ile - Gln - Asn - Cys - Pro - Leu - Gly - $NH_2$ |

Table 2

| Reaction Specificity of Enzyme | | |
|---|---|---|
| Peptide | Retention time (min.) | % Cleavage |
| 1. α-MF<br>dynorphin | 22.38<br>19.12 | not less than 90%<br>not less than 90% |
| 2. neurotensin<br>tuftsin | 19.60<br>5.00 | 0<br>0 |
| 3. β-endorphin<br>α-hamp<br>oxytocin<br>CRF | 21.22<br>18.44<br>18.35<br>19.81 | 0<br>0<br>0<br>0 |

(4) Response to Various Inhibitors

Using the purified enzyme described in Example 2-(3) and 1.5 µg of chemically synthesized α-MF as a substrate, the effects of various inhibitors on the enzymatic activity was studied in 0.05 M acetate buffer (pH 4.0). Table 3 shows the results as to whether the enzymatic reaction was inhibited or not together with the tested inhibitors and their concentration. The peptidase of the present invention was not inhibited by any of the inhibitors tested.

Table 3

| Effects of Protease Inhibitors on Enzymatic Activity | | |
|---|---|---|
| Inhibitor | Concentration | Inhibition |
| monoiodoacetic acid | $2 \times 10^{-4}$ M | -* |
| PMSF | $2 \times 10^{-4}$ M | - |
| PCMB | $2 \times 10^{-4}$ M | - |
| TLCK | $1 \times 10^{-3}$ M | - |
| DFP | $2 \times 10^{-4}$ M | - |
| Leupeptin | $1 \times 10^{-4}$ M | - |
| EDTA | $1 \times 10^{-3}$ M | - |
| Pepstatin | $1 \times 10^{-5}$ M | - |
| Control | | - |

* - indicates that the reaction was not inhibited

**Claims**

**1.** A peptidase secreted by yeast, characterized in that:

(l) it cleaves the bond in amino acid sequence of ... Leu -Lys ... in a peptide chain specifically at the C-terminal side of Leu residue;

(2) it is adsorbed by DEAE cellulose or DEAE dextran, and eluted by 0.l - 0.6 M NaCl;

(3) it has molecular weight of ca. l00,000 estimated from silica gel molecular sieve chromatography, and of ca. 750,000 estimated from Sephacryl® molecular sieve chromatography;

(4) it consists subunits of ca. 55 kd and ca. 63 kd when determined by SDS-PAGE;

(5) its optimum pH for activity is 3 - 4.5;

(6) its optimum temperature for activity is 35 - 42° C; and

(7) its activity is not inhibited by any of the following: monoiodoacetic acid, phenylmethylsulfonyl fluoride (PMSF), p-chloro-mercuribenzoic acid (PCMB), or diisopropyl fluorophosphate (DFP) in a concentration of $2 \times l0^{-4}$ M, tosyl - L - lysin chloromethyl ketone (TLCK) and EDTA at $l \times l0^{-3}$ M, leupeptin at $l \times l0^{-4}$ M, and pepstatin at $l \times l0^{-5}$ M.

2. A peptidase according to Claim I wherein it acts on an $\alpha$-factor of the yeast, Trp - His - Trp - Leu - Gln - Leu - Lys - Pro - Gly - Gln - Pro - Met - Tyr to give Trp - His - Trp - Leu - Gln - Leu and Lys - Pro - Gly - Gln - Pro -Met - Tyr.

3. A peptidase according to Claim I wherein it is secreted by Saccharomyces yeast.

4. A process for preparation of a peptidase secreted by yeast, which comprises immobilizing Saccharomyces yeast for culture on an immobilizing support in high density such that the peptidase is recovered from the culture supernatant, said peptidase being characterized in that:
(1) it cleaves the bond in amino acid sequence of ... Leu - Lys ... in a peptide chain specifically at the C-terminal side of Leu residue;
(2) it is adsorbed by DEAE cellulose or DEAE dextran, and eluted by 0.1 - 0.6 M NaCl;
(3) it has molecular weight of ca. 100,000 estimated from silica gel molecular sieve chromatography, and of ca. 750,000 estimated from Sephacryl® molecular sieve chromatography;
(4) it consists of subunits having ca. 55 kd and ca. 63 kd when determined by SDS-PAGE;
(5) its optimum pH for activity is 3 - 4.5;
(6) its optimum temperature for activity is 35 - 42° C; and
(7) its activity is not inhibited by any of the following: monoiodoacetic acid, phenylmethylsulfonyl fluoride (PMSF), p-chloro-mercuribenzoic acid (PCMB), or diisopropyl fluorophosphate (DFP) in a concentration of 2 x $10^{-4}$ M, tosyl - L - lysin chloromethyl ketone (TLCK) and EDTA at 1 x $10^{-3}$ M, leupeptin at 1 x $10^{-4}$ M, and pepstatin at 1 x $10^{-5}$ M.

5. A process of Claim 4 wherein the immobilizing support is a hydrophilic photo-crosslinkable resin.

6. A process according to Claim 4 wherein the yeast is immobilized in such density that 1 - 2 g of yeast is immobilized on 1 g of an immobilizing support.

7. A process according to Claim 4 wherein the peptidase is purified by DEAE cellulose or DEAE dextran support and by molecular sieve support, from the supernatant of a culture.

**Revendications**

1. Peptidase sécrétée par une levure, caractérisée en ce que :
(1) elle clive la liaison d'une séquence d'acides aminés ... Leu - Lys... qui se trouve au sein d'une chaîne peptidique spécifiquement à l'extrémité carboxyle du résidu Leu ;
(2) elle est adsorbée par la DEAE cellulose ou le DEAE dextrane et éluée par du NaCl 0,1 à 0,6 M ;
(3) elle a un poids moléculaire d'environ 100.000, si elle est estimée par chromatographie sur tamis moléculoire de gel de silice et d'environ 750.000 si elle est estimée par chromatographie sur tamis moléculaire de Sephacryl® ;
(4) elle est constituée de sous unités de 55 et 63 kd, environ, d'après l'estimation effectuée en SDS-PAGE ;
(5) son pH d'activité optimale se situe entre 3 et 4,5 ;
(6) sa température d'activité optimale se situe entre 35° C-42° C ; et
(7) son activité n'est inhibée par aucun des produits suivants : acide monoidoacétique, fluorure de phényleméthylesulfonyle (PMSF), acide p-chloro-mercuribenzoïque (PCMB) ou fluorophosphate de diisopropyle (DFP) à une concentration de 2 x $10^{-4}$ M, tosyl- L - lysine chlorométhyl-cétone (TLCK) et EDTA 1 x $10^{-3}$ M, leupeptine 1 x $10^{-4}$ M et pepstatine 1 x $10^{-5}$ M.

2. Peptidase selon la revendication 1, qui agit sur un facteur alpha de la levure dont la structure est la suivante : Trp - His - Trp - Leu - Gln - Leu - Lys - Pro - Gly - Gln - Pro - Met - Tyr pour former : Trp - His - Trp - Leu - Gln - Leu et Lys - Pro - Gly - Gln - Pro - Met - Tyr.

3. Peptidase selon la revendication 1, sécrétée par la levure Saccharomyces.

4. Procédé de préparation d'une peptidase sécrétée par une levure, qui comporte l'immobilisation de levure Saccharomyces pour en faire une culture sur un support d'immobilisation à haute densité, de telle sorte que la peptidase soit recueillie dans le surnageant de la culture, ladite peptidase étant caractérisée en ce que :

(1) elle clive la liaison d'une séquence d'acides aminés... Leu - Lys... qui se trouve au sein d'une chaîne peptidique spécifiquement à l'extrémité carboxyle du résidu Leu ;
(2) elle est adsorbée par la DEAE cellulose ou le DEAE dextrane et éluée par du NaCl 0,1 à 0,6 M ;
(3) elle a un poids moléculaire d'environ 100.000, si elle est estimée par chromatographie sur tamis moléculaire de gel de silice et d'environ 750.000 si elle est estimée par chromatographie sur tamis moléculaire de Sephacryl® ;
(4) elle est constituée de sous unités de 55 et 63 kd, environ, d'après l'estimation effectuée en SDS-PAGE ;
(5) son pH d'activité optimale se situe entre 3 et 4,5 ;
(6) sa température d'activité optimale se situe entre 35° C et 42° C et son activité n'est inhibée par aucun des produits suivants : acide monoiodoacétique, fluorure de phényleméthylesulfonyle (PMSF), acide p-chloro-mercuribenzoïque (PCMB) ou fluorophosphate de diisopropyle (DFP) à une concentration de 2 x $10^{-4}$ M, tosyl - L - lysine chlorométhyl-cétone (TLCK) et EDTA
1 x $10^{-3}$ M, leupeptine 1 x $10^{-4}$ M et pepstatine 1 x $10^{-5}$ M.

**5.** Procédé selon la revendication 4 dans lequel le support immobilisant est une résine hydrophyle photoréticulable.

**6.** Procédé selon la revendication 4 dans lequel la levure est immobilisée à une densité telle que 1 - 2g de levure est immobilisé dans 1 g de support d'immobilisation.

**7.** Procédé selon la revendication 4 dans lequel la peptidase est purifiée sur un support constitué par de la DEAE cellulose ou du DEAE dextrane et sur un support constitué par un tamis moléculaire, à partir du surnageant de culture.

**Patentansprüche**

**1.** Peptidase, die von Hefe ausgeschieden wird, dadurch **gekennzeichnet,** dass

(1) sie die Bindung ...Leu-Lys... in der Aminosäuresequenz in einer Peptidkette spezifisch an der C-terminalen Seite des Leu-Restes spaltet;
(2) sie an DEAE-Zellulose oder DEAE-Dextran adsorbiert wird und durch 0,1 bis 0,6 M NaCl eluiert wird;
(3) sie ein Molekulargewicht von ca. 100.000, bestimmt durch Kieselgel-MolekularsiebChromatografie, und ca. 750.000, bestimmt durch Sephacryl$^{R}$-Molekularsieb-Chromatografie aufweist;
(4) sie aus Untereinheiten von ca. 55 kd und ca. 63 kd, bestimmt durch SDS-PAGE, besteht;
(5) ihr pH-Optimum der Aktivität bei 3 bis 4,5 liegt;
(6) ihr Temperaturoptimum der Aktivitätt bei 35 bis 42° C liegt; und
(7) ihre Aktivität durch keine der folgenden Substanzen inhibiert wird: Monojodessigsäure, Phenylmethylsulfonylfluorid (PMSF), p-Chlor-mercuribenzoesäure (PCMB) oder Diisopropylfluorphosphat (DFP) in einer Konzentration von 2 x $10^{-4}$ M, Tosyl-L-lysinchlormethylketon (TLCK) und EDTA bei 1 x $10^{-3}$M, Leupeptin bei 1 x $10^{-4}$ M und Pepstatin bei 1 x $10^{-5}$ M.

**2.** Peptidase nach Anspruch 1, die auf einen alpha-Faktor der Hefe,
Trp-His-Trp-Leu-Gln-Leu-Lys-Pro-Gly-Gln-Pro-Met-Tyr, unter Erhalt von Trp-His-Trp-Leu-Gln-Leu und Lys-Pro-Gly-Gln-Pro-Met-Tyr einwirkt.

**3.** Peptidase nach Anspruch 1, die aus Saccharomyces-Hefe ausgeschieden wird.

**4.** Verfahren zur Herstellung einer Peptidase, die aus Hefe ausgeschieden wird, umfassend die Immobilisierung von Saccharomyces-Hefe für die Kultur auf einem Immobilisierungsträger in hoher Dichte, so dass die Peptidase aus dem Kulturüberstand gewonnen wird, wobei die Peptidase dadurch
**gekennzeichnet** ist, dass

(1) sie die Bindung ...Leu-Lys... in der Aminosäuresequenz in einer Peptidkette spezifisch an der C-terminalen Seite des Leu-Restes spaltet;
(2) sie an DEAE-Zellulose oder DEAE-Dextran adsorbiert wird und durch 0,1 bis 0,6 M NaCl eluiert wird;

(3) sie ein Molekulargewicht von ca. 100.000, bestimmt durch Kieselgel-Molekularsieb-Chromatografie, aufweist, und ca. 750.000, bestimmt durch Sephacryl$^{R}$-Molekularsieb-Chromatografie, aufweist;
(4) sie aus Untereinheiten von ca. 55 kd und ca. 63 kd, bestimmt durch SDS-PAGE, besteht;
(5) ihr pH-Optimum der Aktivität bei 3 bis 4,5 liegt;
(6) ihr Temperaturoptimum der Aktivität bei 35 bis 42° C liegt; und
(7) ihre Aktivität durch keine der folgenden Substanzen inhibiert wird: Monojodessigsäure, Phenylmethylsulfonylfluorid (PMSF), p-Chlor-mercuribenzoesäure (PCMB) oder Diisopropylfluorphosphat (DFP) in einer Konzentration von 2 x $10^{-4}$ M, Tosyl-L-lysinchlormethylketon (TLCK) und EDTA bei 1 x $10^{-3}$M, Leupeptin bei 1 x $10^{-4}$ M und Pepstatin bei 1 x $10^{-5}$ M.

**5.** Verfahren nach Anspruch 4, worin der Immobilisierungsträger ein hydrophiles fotovernetzbares Harz ist.

**6.** Verfahren nach Anspruch 4, worin die Hefe in einer solchen Dichte immobilisiert wird, dass 1 bis 2 g Hefe auf 1 g Immobilisierungsträger immobilisiert werden.

**7.** Verfahren nach Anspruch 4, worin die Peptidase durch DEAE-Zellulose oder einem DEAE-Dextranträger und durch einen Molekularsiebträger aus dem Überstand der Kultur gereinigt wird.

Fig. 1

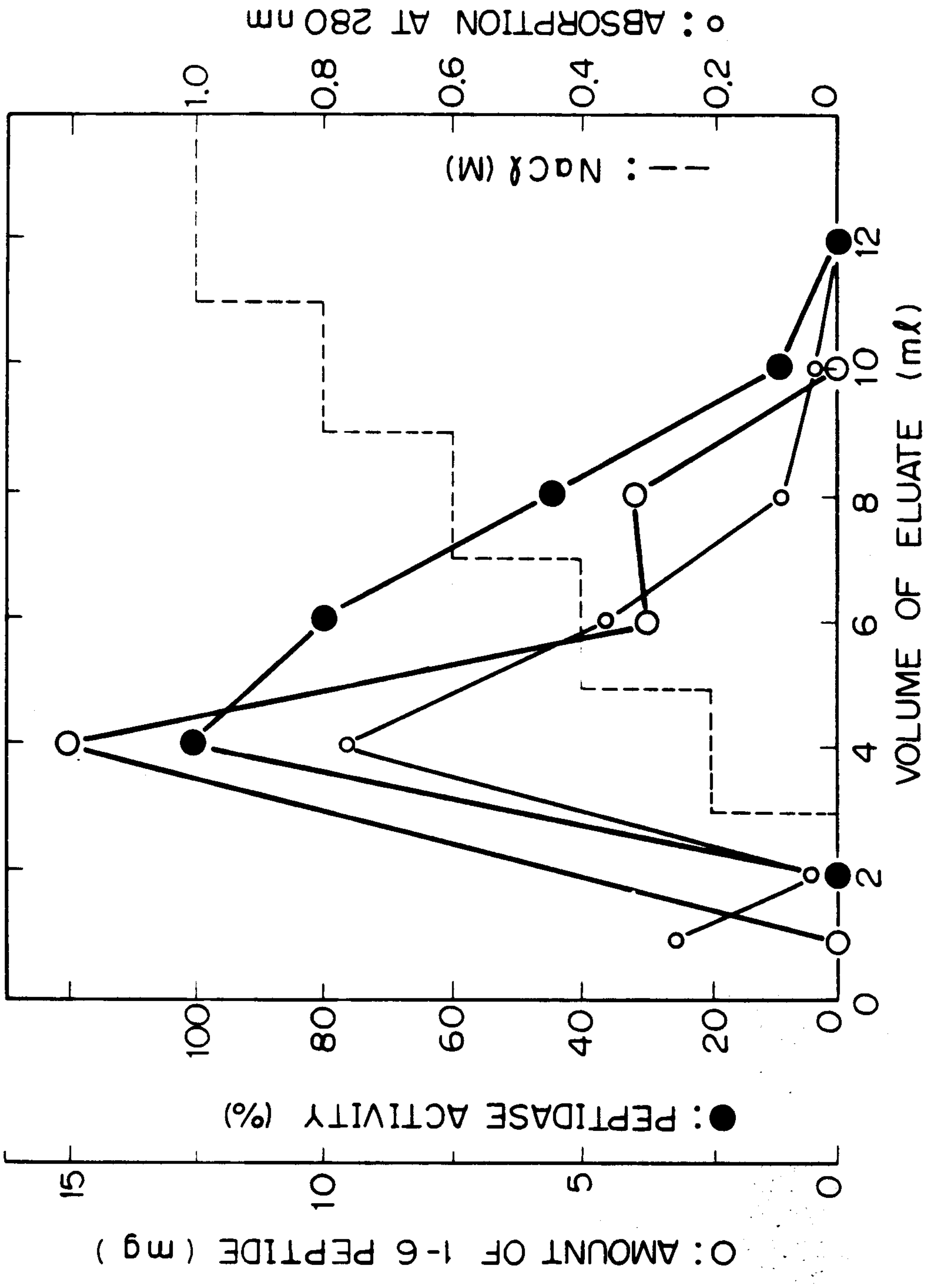
○: AMOUNT OF 1-6 PEPTIDE (mg)
●: PEPTIDASE ACTIVITY (%)
o: ABSORPTION AT 280 nm
--- : NaCl (M)
VOLUME OF ELUATE (ml)
0
5
10
15
0
20
40
60
80
100
0
0.2
0.4
0.6
0.8
1.0
0
2
4
6
8
10
12

Fig. 2

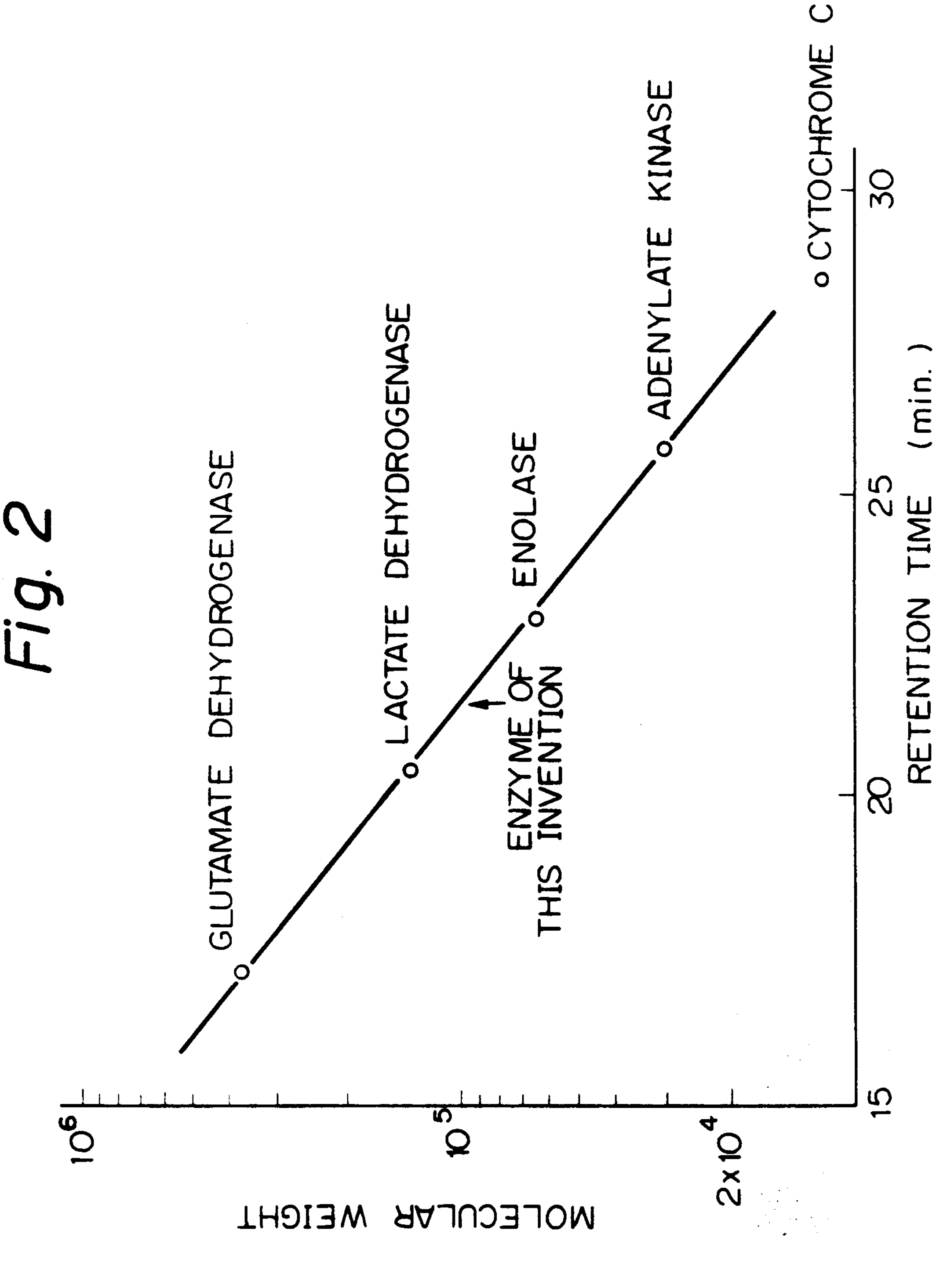
GLUTAMATE DEHYDROGENASE
LACTATE DEHYDROGENASE
ENZYME OF THIS INVENTION
ENOLASE
ADENYLATE KINASE
CYTOCHROME C
MOLECULAR WEIGHT
$10^6$
$10^5$
$2\times10^4$
15
20
25
30
RETENTION TIME (min.)

Fig. 3

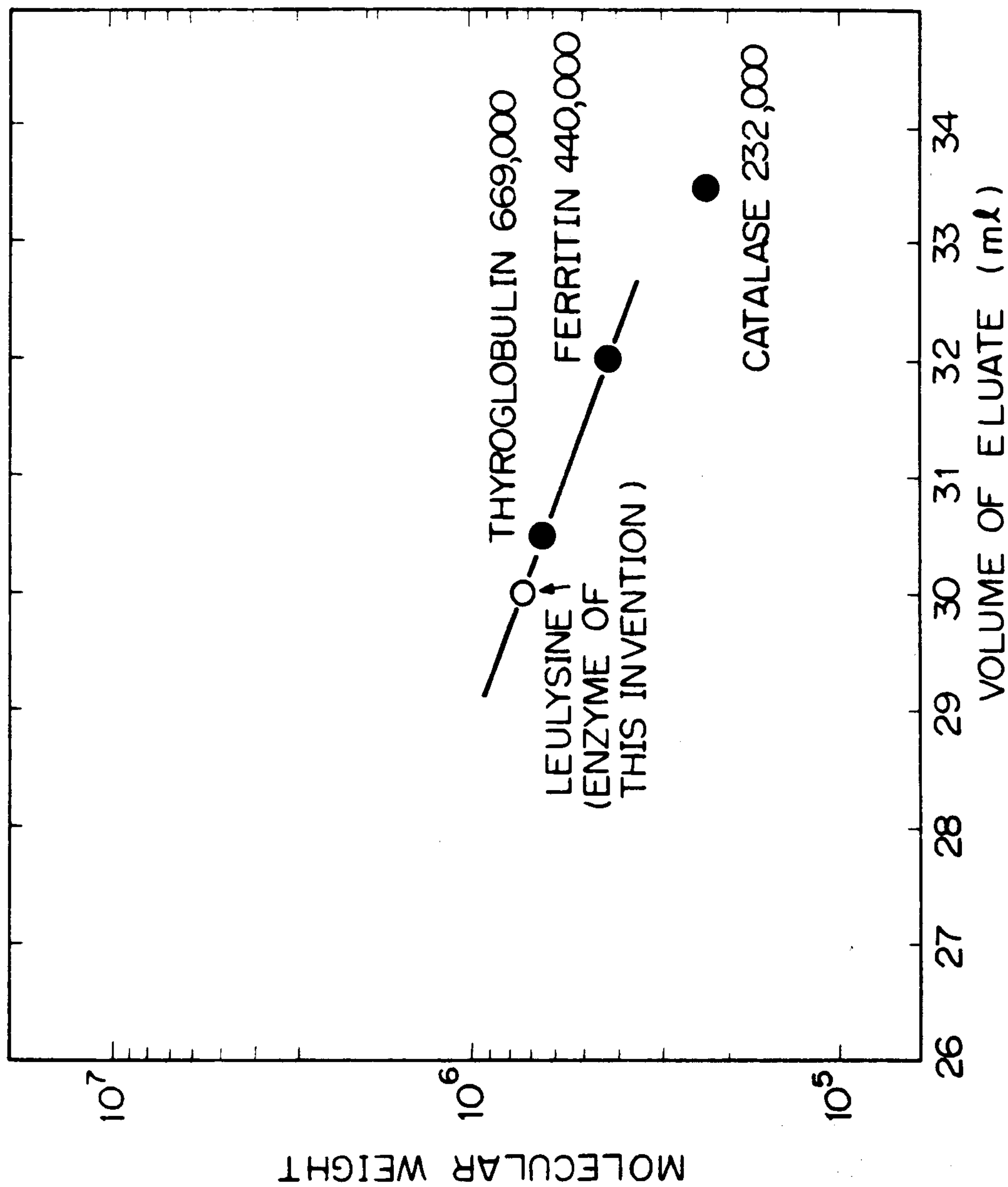
THYROGLOBULIN 669,000
FERRITIN 440,000
CATALASE 232,000
LEULYSINE (ENZYME OF THIS INVENTION)
VOLUME OF ELUATE (mℓ)
MOLECULAR WEIGHT
26
27
28
29
30
31
32
33
34
$10^7$
$10^6$
$10^5$

Fig. 4

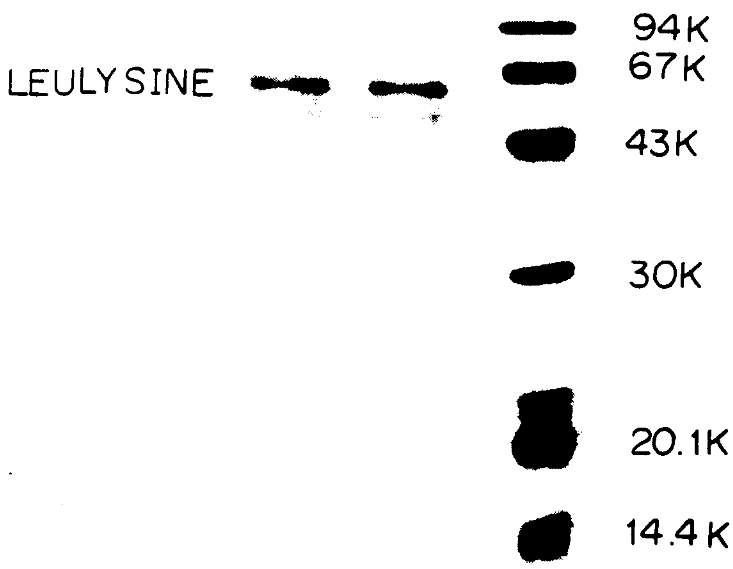


SDS-PAGE OF
THIS ENZYME

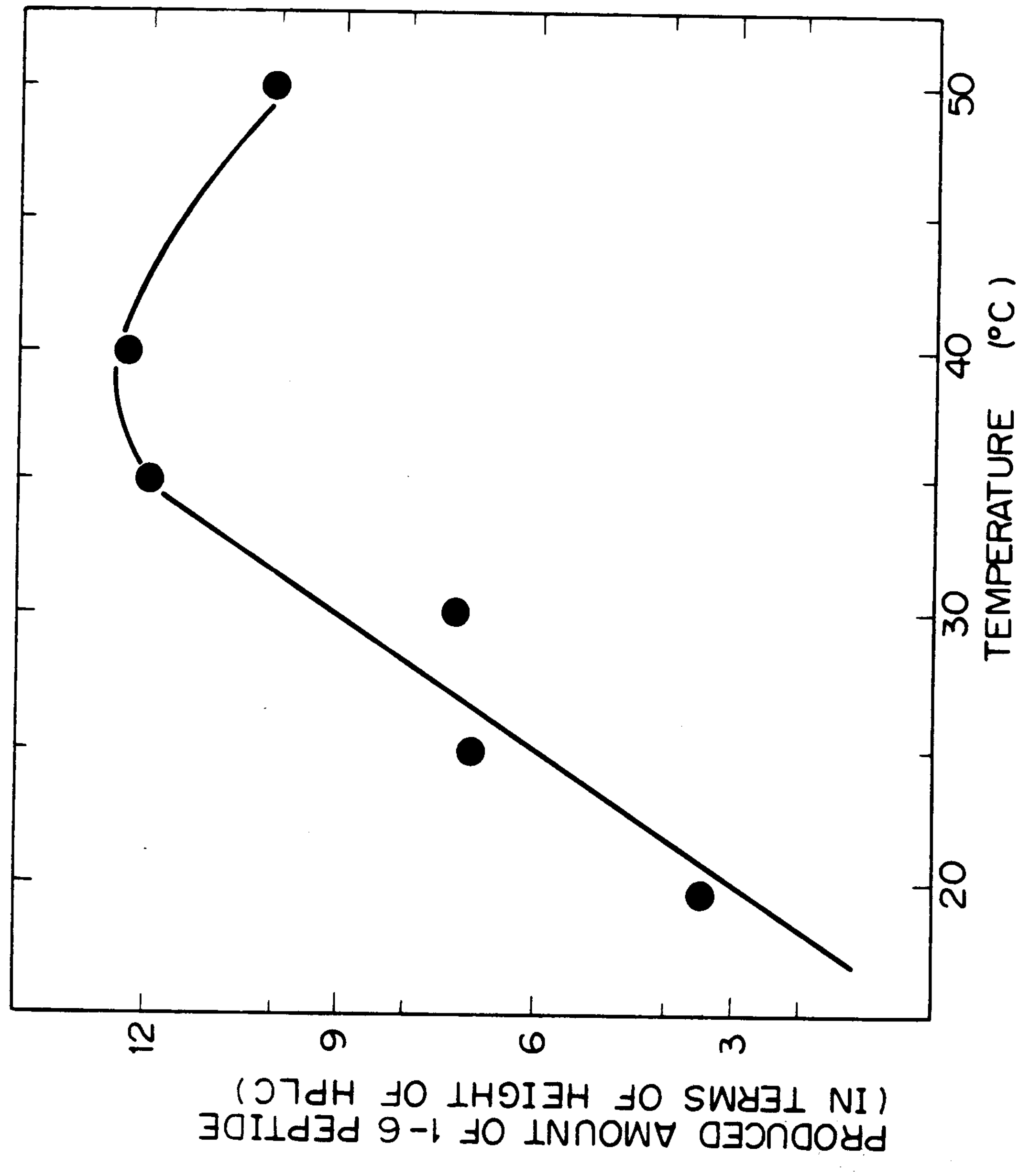

PRODUCED AMOUNT OF 1-6 PEPTIDE
(IN TERMS OF HEIGHT OF HPLC)
12
9
6
3
20
30
40
50
TEMPERATURE (°C)


Fig. 5

Fig. 6